# EUROPEAN PATENT SPECIFICATION

(11) **EP 4 590 218 B1**
(45) Date of publication and mention of the grant of the patent: **23.09.2026**
(21) Application number: 23754198.2
(22) Date of filing: 03.08.2023
(51) Int. Cl.: A61B 18/14, A61B 34/30, A61B 18/18

(54) **JOINT AND ARTICULATION MECHANISM**
GELENK UND GELENKMECHANISMUS
CHARNIÈRE ET MÉCANISME D'ARTICULATION

(30) Priority: 23.09.2022 GB 202213947
(43) Date of publication of application: 30.07.2025
(73) Proprietor: Creo Medical Limited, Chepstow Gwent NP16 5UH (GB)
(72) Inventor: TURNER, Louis, Chepstow (GB); JONES, Warren, Chepstow (GB); ULLRICH, George, Chepstow (GB); HANCOCK, Christopher, Chepstow (GB); FITZSIMONS, Duncan Foster, London Ltd. Pendragon House 65 London Road St Albans Hertfordshire AL1 1LJ (GB)
(74) Representative: Mewburn Ellis LLP
(86) International application number: PCT/EP2023/071590
(87) International publication number: WO 2024/061525

(56) References cited:
- EP-A1- 3 420 937
- EP-A1- 3 539 495
- WO-A1-2021/157138
- WO-A1-2023/052781
- US-A1- 2007 184 682
- US-A1- 2010 116 078
- US-A1- 2014 249 557
- US-A1- 2018 280 084
- US-A1- 2021 006 023

## Description

### Field of the Invention

The present invention relates to a joint and an articulation mechanism including a joint and particularly, although not exclusively, to a joint and an articulation mechanism including an electrical pathway through the joint for conveying electromagnetic energy (e.g. radiofrequency RF energy and/or microwave frequency energy). The joint and articulation mechanism may find particular application in robotic surgery.

### Background

It is known to provide electrosurgical instruments with joints and/or articulation mechanisms to steer and manipulate a distal end of the instrument for correct positioning of the instrument tip. Mechanisms are also known to convey electromagnetic energy across such joints to deliver the energy to the instrument tip.

It may be useful in some circumstances to provide articulation mechanisms with a reduced footprint, for example to enable use of an electrosurgical instrument in a confined space. The confined space may be a confined space within a system for positioning the instrument (such as a manually or robotically operated locating system), or within the space to be treated (such as a particular body cavity or body passage).

The present invention has been devised in light of the above considerations.

US2010/0116078A1 discloses a robot joint having an electricity connecting device.

### Summary of the Invention

The invention is defined by the appended independent claims 1 and 8. The appended dependent claims define optional features of the invention.

### Summary of the Disclosure

At a general level, there is provided a joint for an articulation mechanism. The joint includes an electrical pathway that is arranged to convey electromagnetic energy through the moving parts of the joint, thereby reducing the need for separate electrical connections alongside the joint. That is, the same physical structures that move with respect to each other to enable the mechanical function of the joint also provide a pathway for electromagnetic energy. There is also provided an articulation mechanism including such joints, and an electrosurgical device including an electrosurgical instrument connected to such an articulation mechanism.

According to a first aspect of the disclosure, there is provided a joint for an articulation mechanism for use in electrosurgery. The joint comprises a first electrically insulating planar substrate having a first surface and a second surface opposite the first surface, and a second electrically insulating planar substrate having a first surface and a second surface opposite the first surface. The second planar substrate is mounted to be rotatable about a pivot point with respect to the first planar substrate such that the first surface of the first planar substrate is in contact with the second surface of the second planar substrate.

In an embodiment, the pivot point represents a rotational axis passing through each of the first and second planar substrates and extending perpendicularly to the plane of the planar substrates.

The joint further comprises a first conductive element arranged on the first surface of the first planar substrate and a second conductive element arranged on the second surface of the second planar substrate. The first and second conductive elements are arranged such that the first and second conductive elements form a first electrical pathway through the joint.

In an embodiment, the joint may be or form part of a mechanical wrist or a robotic wrist for use in electrosurgery. For example, the joint may be configured in use to couple to or be held by a robotic surgical system, for example, at a distal end of a robotic arm of the robotic surgical system.

Providing a joint having an electrical pathway of this type may reduce the overall size or footprint of the joint by reducing space required to provide an electrical connection across the joint. An electrical pathway can be housed within the joint, between the moving elements of the joint (the planar substrates). That is, the same physical structures that move with respect to each other to enable the mechanical function of the joint (e.g. the two planar substrates having the two conductive elements) also provide a pathway for electromagnetic energy. That is, the first electrical pathway is integral with the physical elements (e.g. the two planar substrates) of the joint.

For example, the first conductive element may be used to convey energy, via the first electrical pathway, away from the joint in one direction (e.g. distally), whereas the second conductive element may be used to convey energy, via the first electrical pathway, away from the joint in another direction (e.g. proximally).

In some arrangements, the first conductive element and the second conductive element may be in direct electrical contact. The first conductive element and the second conductive element may be configured to slide against one another to maintain direct electrical contact when the second planar substrate is rotated with respect to the first planar substrate. An electrical connection of this type, between conductive elements arranged on two contacting surfaces that slide with respect to each other can provide greater flexibility in the joint, since (unlike what might happen with a separate electrical connection), the electrical connection does not need to bend, twist, or flex as the joint rotates.

In some arrangements, at least one of the first conductive element and the second conductive element may be a conductive coating applied to the respective surface of the respective planar substrate. This provides a particular implementation of a conductive element for use in a joint according to the first aspect. For example, the conductive coatings may not exist independently of the planar substrates, but instead be coatings disposed on the planar substrates.

In some arrangements, the joint may further comprise a second electrical pathway through the joint, the second electrical pathway being separate from the first electrical pathway. Providing a second electrical pathway separate from the first electrical pathway can enable the joint to provide a bipolar electrical connection, as may be required, for example, for certain configurations of electrosurgical instruments. For example, the bipolar electrical connection can be used to convey radiofrequency electromagnetic (EM) energy and/or microwave frequency EM energy.

In some arrangements, the joint may further comprise a third conductive element arranged on the first surface of the second plate, and an electrically conductive bracket mounted in contact with the first surface of the second plate. The third conductive element and the bracket may thereby form the second electrical pathway through the joint. The bracket may extend across the pivot point or rotational axis, and may be configured to hold the first and second plates together. For example, the bracket may be arranged to at least partially surround an overlapping region of the first and second insulating planar substrates such that the rotational axis of the pivot point extends through the bracket. The bracket may have a substantially C-shape in cross-section to wrap around the first and second planar substrates and, for example, bear against the second surface of the first planar substrate and the first surface of the second planar substrate. For example, the third conductive element may be used to convey energy, via the second electrical pathway, away from the joint in one direction (e.g. distally), whereas the bracket may be used to convey energy, via the second electrical pathway, away from the joint in another direction (e.g. proximally). This provides a particular implementation to provide a dual or bipolar electrical connection through or across the joint.

In some arrangements, at least one of the bracket and the first planar substrate may be integrally formed with a part of an electrosurgical instrument. Where the joint is to be used to steer or articulate an electrosurgical instrument, integrating the joint with the instrument in this way may further reduce the footprint or size of the jointed portion.

In some arrangements, the electrosurgical instrument may comprise a first jaw element and a second jaw element pivotably mounted with the first jaw element. The second jaw element may be pivotably mounted to rotate or pivot about a second pivot point separate from the pivot point of the joint. In other words, the rotational axis about which the second jaw element rotates may be different from the rotational axis of the joint. At least one of the bracket and the first planar substrate may be integrally formed with the first jaw element. This may provide a particular implementation of an electrosurgical instrument having a pair of jaws and including a joint for articulating the instrument.

In some arrangements, the electrosurgical instrument may comprise a radiating structure for delivering electromagnetic energy (e.g. RF EM energy and/or microwave frequency EM energy) received from the first and second electrical pathways through the joint to biological tissue, for example, located in a gap between the first and second jaws. For example, the radiating structure may comprise a first electrode connected to the first electrical pathway and a second electrode connected to the second electrical pathway for delivering electromagnetic issue to tissue. The first and second electrodes may form part of an energy delivery system for delivering EM energy into the gap between the first and second jaws.

In some arrangements, each of the first electrical pathway and the second electrical pathway may be arranged to convey electromagnetic energy (e.g. RF energy and/or microwave frequency energy) from an electrosurgical generator through the joint. For example, the joint may comprise a connector (or terminal) for connecting the first and second electrical pathways to a power cable (or feed structure) connected to the electrosurgical generator. In this way, the joint may be configured to provide power to an electrosurgical instrument from an electrosurgical generator. Such electrosurgical generators are known, e.g. as disclosed in WO 2012/076844.

In some arrangements, the connector may be configured to connect to a coaxial cable as the power cable, the coaxial cable including an inner conductor and an outer conductor separated by a dielectric layer. The connector may include a first terminal for connecting the inner connector to the first electrical pathway and a second terminal for connecting the outer conductor to the second electrical pathway. This can enable the joint to be compatible with a coaxial power cable, as may be required for particular system configurations.

In some arrangements, the connector may be configured to connect to a microstrip as the power cable, the microstrip including first and second conductors separated by a planar dielectric layer, e.g., a flexible planar dielectric layer or ribbon. The connector may include a first terminal for connecting the first conductor to the first electrical pathway and a second terminal for connecting the second conductor to the second electrical pathway. This can enable the joint to be compatible with a microstrip power cable, as may be required for particular system configurations.

In some arrangements, at least one of the conductive elements may be located in a recess in the respective surface of the respective planar substrate. For example, the recess may have a corresponding (e.g. the same) depth as a thickness of the conductive element, such that the conductive element is arranged to be substantially flush with the surface of the planar substrate. This can enable the provision of an electrical connection while reducing friction or wear that may otherwise be caused by movement of conductive elements that project from a surface of a planar substrate.

In some arrangements, at least one of the first planar substrate and the second planar substrate comprises a circular portion about the pivot point. For example, the circular portion may be centred about the pivot point or rotational axis. This can enable increased ease of rotation of the planar substrate by providing the substrate with a constant radial extent from the pivot point about at least part of the planar substrate.

In some arrangements, the joint may comprise a circular bore at a central position of the circular portion. The circular bore may form the pivot point for the circular portion. For example, the joint may include a pin or axle passing through the circular bore to form the pivot point of the joint. The pin or axle may be flared at one or both ends to hold it in the bore.

In some arrangements, the conductive element of the respective planar substrate may form a ring around the circular bore. This may enable the respective conductive elements to remain in contact as the planar substrate is rotated or pivoted to articulate the joint.

In some arrangements, at least one of the first planar substrate and the second planar substrate may have a keyhole shape, comprising a circular portion about the pivot point and a rectangular portion (or tab portion) extending from the circular portion. The rectangular portion may provide an attachment or connection point for connecting an external electrical connection (such as a connection from a power cable or to a feed structure) to the joint.

In some arrangements, each of the first planar substrate and the second planar substrate may have a keyhole shape. The rectangular portion of the first planar substrate may not overlap with the rectangular portion of the second planar substrate. This can enable electrical connections to be made to each of the first and second planar substrates, while isolating the connection points from each other such that the electrical connection or pathway through the joint is only via the electrical pathways of the joint. In other words, it may avoid a short circuit across the joint between the incoming and outgoing power connections, which may otherwise cause inconsistent delivery of electromagnetic energy through or across the joint. In an embodiment, an abutment portion may overlay the rectangular portion of one of the first and second planar substrates and have a stop surface configured in use to abut the rectangular portion of the other of the first and second planar substrates in order to define a limit of rotational movement between the first and second planar substrates so as to avoid a short circuit. The abutment portion may be held in place by the bracket.

According to a second aspect of the disclosure, there is provided an articulation mechanism for steering an electrosurgical instrument, the articulation mechanism having a proximal end and a distal end. The articulation mechanism comprises a first terminal located at the proximal end of the articulation mechanism for receiving electromagnetic energy (e.g. RF energy and/or microwave frequency energy) from an electrosurgical generator and a second terminal located at the distal end of the articulation mechanism for delivering the electromagnetic energy received from the electrosurgical generator to the electrosurgical instrument, a first rotating joint having a first axis of rotation, and a second rotating joint arranged distally of the first rotating joint and having a second axis of rotation different from the first axis of rotation. In other words, the second rotating joint is connected to a distal end of the first rotating joint, such that articulating the first rotating joint moves the location of the second joint about the axis of rotation of the first joint. By adjusting a rotation angle of the first joint and the second joint, a tip of an attached electrosurgical instrument can be steered through a range of angles.

The first and second rotating joints each comprise a first joint element having a first conductive element on a surface thereof, and a second joint element having a second conductive element on a surface thereof. Each second joint element is rotatably mounted with respect to each first joint element such that the surface of the first joint element having the conductive element is in contact with the surface of the second joint element having the conductive element. In this way, the first and second conductive elements form a first electrical pathway through the joint. As with the first aspect, this enables the provision of an articulation mechanism having joints of reduced footprint and greater joint flexibility.

In an embodiment, the articulation mechanism may be or form part of a mechanical wrist or a robotic wrist for use in electrosurgery. For example, the articulation mechanism may be configured in use to couple to or be held by a robotic surgical system, for example, at a distal end of a robotic arm of the robotic surgical system.

For example, the first conductive element may be used to convey energy, via the first electrical pathway, away from the articulation mechanism in one direction (e.g. distally), whereas the second conductive element may be used to convey energy, via the first electrical pathway, away from the articulation mechanism in another direction (e.g. proximally).

In some arrangements, the first and second axes of rotation may be perpendicular to one another. This provides a particular arrangement of the articulation mechanism that may enable a greater range of steering positions. It may also enable increased ease of control by allowing, for example, pitch of an attached electrosurgical instrument to be controlled by one of the joints, and yaw of an attached electrosurgical instrument to be controlled by the other of the joints.

In some arrangements, the first and second joints may each comprise a second electrical pathway separate from the first electrical pathway. This can enable the articulation mechanism to provide a bipolar electrical connection through the joints, as may be required, for example, for certain configurations of electrosurgical instruments to be mounted to the articulation mechanism. For example, the bipolar electrical connection can be used to convey radiofrequency EM energy and/or microwave frequency EM energy.

In some arrangements, one of the first and second joint elements of each of the first and second joints may include a third conductive element on a second surface thereof, the third conductive element forming part of the second electrical pathway through each joint. Additionally, in some arrangements, the other of the first and second joint elements of each of the first and second joints may include a fourth conductive element on a second surface thereof, the fourth conductive element forming part of the second electrical pathway through each joint. This provides a particular configuration for the second electrical pathway of the articulation mechanism.

For example, the third conductive element may be used to convey energy, via the second electrical pathway, away from the articulation mechanism in one direction (e.g. distally), whereas the fourth conductive element may be used to convey energy, via the second electrical pathway, away from the articulation mechanism in another direction (e.g. proximally). In some arrangements, the first rotating joint may be directly connected to the second rotating joint with no intervening elements.

In some arrangements, the first rotating joint and the second rotating joint may be connected via a connection comprising a coaxial cable. The coaxial cable may represent a physical connection in addition to an electrical connection, such that a proximal end of the second rotating joint is physically attached to a distal end of the first rotating joint via the connection including the coaxial cable.

In some arrangements, at least one of the first rotating joint and the second rotating joint may be a coaxial rotating joint comprising a first coaxial cable and a second coaxial cable, each coaxial cable comprising an inner conductor and an outer conductor separated by a dielectric layer.

The first coaxial cable may form the first joint element. The, or each, joint may further include a first conductive sleeve forming part of the first electrical pathway, and arranged to form a rotatable electrical connection between the respective inner conductors. For example, the inner conductors may be configured to extend beyond the dielectric layers and the outer conductors to engage with the sleeve. The first conductive sleeve may thereby form the second joint element.

The, or each, coaxial joint may further include a second conductive sleeve forming part of the second electrical pathway, and arranged to form a rotatable electrical connection between the respective outer conductors. For example, the second conductive sleeve may overlap the respective outer conductors to establish an electrical connection therewith.

A coaxial joint of this type may enable an articulation mechanism with a reduced footprint by providing a joint that extends along, and rotates about, an axial direction of a connected coaxial cable.

In some examples, at least one of the first conductive sleeve and the second conductive sleeve is freely rotatable with respect to each of the first coaxial cable and the second coaxial cable. In other words, the first conductive sleeve and/or the second conductive sleeve may not be fixed to either of the first or second coaxial cables, and may be free to rotate with respect to each of the inner or outer conductors of the first coaxial cable and the second coaxial cable. Alternatively, the first conductive sleeve and/or the second conductive sleeve may be fixed to only one of the first coaxial cable and second coaxial cable, and may thereby rotate in unison with the one coaxial cable with respect to the other coaxial cable.

In some arrangements, the coaxial rotating joint may further comprise an electrically insulating sleeve arranged between the first conductive sleeve and the second conductive sleeve. The electrically insulating sleeve insulates the two conductive sleeves from each other, and also insulates the first electrical pathway from the second electrical pathway. The insulating sleeve may be free to rotate with respect to each of the first coaxial cable and the second coaxial cable. Alternatively, the insulating sleeve may be attached to one of the first coaxial cable and the second coaxial cable and may thereby rotate in unison with the first coaxial cable or the second coaxial cable. The insulating sleeve may be free to rotate with respect to each of the first conductive sleeve and the second conductive sleeve. Alternatively, the insulating sleeve may be attached to at least one of the first conductive sleeve and the second conductive sleeve. In some further arrangements, the insulating sleeve may be attached to both of the first conductive sleeve and the second conductive sleeve, and may thereby attach the first conductive sleeve to the second conductive sleeve.

In some arrangements, each of the first rotating joint and the second rotating joint is a coaxial rotating joint. The connection between the first rotating joint and the second rotating joint may comprise one or more bends to introduce an offset of the second axis of rotation compared to the first axis of rotation such that the second axis of rotation is different from the first axis of rotation. For example, since the axis of rotation of a coaxial joint is in an axial direction of the first and second coaxial cables, providing a bend in the connection between the first joint and the second joint may be one way that an offset can be introduced between the respective rotation axes. For example, where the first axis of rotation is perpendicular to the second axis of rotation, the connection may include a right-angle bend, or a plurality of smaller bends that combine to form a right-angle bend.

In some arrangements, the first rotating joint and the second rotating joint may be connected via a connection comprising a microstrip, the microstrip comprising first and second conductors separated by a planar dielectric layer, for example, a flexible planer dielectric layer or ribbon. A microstrip connection may have a smaller lateral extent than a coaxial connection, thereby reducing a footprint or size of the articulation mechanism.

In some arrangements, at least one of the first rotating joint and the second rotating joint may be a joint according to the first aspect, with the first joint element being the first planar substrate and the second joint element being the second planar substrate.

In some arrangements, the articulation mechanism may include a connector for mounting an electrosurgical instrument to the articulation mechanism. This connector may be a physical connection to the electrosurgical instrument to rigidly mount the electrosurgical instrument to a distal end of the articulation mechanism. The connector may further provide an electrical connection between the first and/or second electrical pathways and the electrosurgical instrument, enabling electromagnetic energy (e.g. RF energy and/or microwave frequency energy) to be supplied by the articulation mechanism to the electrosurgical instrument. For example, where the electrosurgical instrument includes a radiating structure comprising a first electrode and a second electrode, the connector may provide a connection between the first electrical pathway and the first electrode and between the second electrical pathway and the second electrode. In an embodiment, the connector comprises first and second terminals, the first terminal for connecting the first electrical pathway to the first electrode, and the second terminal for connecting the second electrical pathway to the second electrode.

In some arrangements, the articulation mechanism may further comprise an electrosurgical instrument integrally formed with the first joint element or the second joint element of the second rotating joint. This may enable a further reduced footprint or size for the articulation mechanism by reducing space required for a connection between the articulation mechanism and the electrosurgical instrument.

In some arrangements, the articulation mechanism may further comprise a sleeve or housing, such as a flexible and electrically insulating sleeve arranged to surround the first and second joints. This sleeve may help shield and/or protect the articulation mechanism from damage. This sleeve may also provide physical protection against shorting from contact with moist tissue or ingress of fluid, and/or maintain consistent contact pressure between moving contact surfaces throughout use.

According to a third aspect of the disclosure, there is provided an electrosurgical device including an articulation mechanism according to the second aspect, and an electrosurgical instrument mounted at the distal end of the articulation mechanism.

According to a fourth aspect of the disclosure, there is provided an electrosurgical instrument comprising an instrument shaft having attached at its distal end the joint of the first aspect and/or the articulation mechanism of the second aspect. A distal end of the joint or articulation mechanism may be attached to a distal end assembly of the instrument for engaging with biological tissue, e.g. for gripping tissue if the distal end assembly contains jaws, and/or for delivering electromagnetic energy (e.g. radio frequency or microwave frequency) to tissue if the distal end assembly has RF electrodes or microwave radiating structures. That is, the joint or articulation mechanism may fit between a distal end of the instrument shaft and a proximal end of the distal end assembly.

The instrument shaft may, for example, contain control wires or rods for articulating the joints and/or elements (e.g. jaws) of the electrosurgical instrument. The instrument shaft may also contain one or more power cables for providing electromagnetic (EM) energy (e.g. radiofrequency RF EM energy and/or microwave frequency EM energy) to the joint or articulation mechanism for conveyance to the distal end assembly. The instrument shaft may contain additional components, such as channels for supplying or extracting gas or fluid (e.g. saline) and/or further control mechanisms (for example for an element such as a retractable blade).

In an embodiment, the electrosurgical instrument of the fourth aspect, the joint of the first aspect and/or the articulation mechanism of the second aspect includes one or more joint mounting brackets mounted to and surrounding all or part of the joint or articulation mechanism. These joint mounting brackets may be electrically isolated from the respective electrical pathways of the joint and articulation mechanism. The joint mounting brackets may be configured to provide a level of physical protection for the joints or articulation mechanism for example against impact or intrusion.

In an embodiment, articulating actuators are provided for the joints or articulating mechanisms. In this example, the joints or actuators include a peg or pulley which may be acted upon by a control wire or rod to articulate the joints. In other embodiments, different control mechanisms or actuators may be used. These may include control mechanisms (such as a servo or motor) and/or other means of conveying an articulating force via a force transfer element from a proximal end of the instrument shaft.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1** illustrates a joint for an articulation mechanism according to an aspect of the present invention;
**Figure 2** illustrates a second view of the joint of Figure 1;
**Figure 3** illustrates a third view of the joint of Figure 1;
**Figure 4** illustrates a fourth view of the joint of Figure 1;
**Figure 5** illustrates a fifth view of the joint of Figure 1;
**Figure 6** illustrates a bracket of the joint of Figure 1;
**Figure 7** illustrates a terminal of the joint of Figure 1;
**Figure 8** illustrates an articulation mechanism according to an aspect of the invention;
**Figure 9** illustrates a second view of the articulation mechanism of Figure 8;
**Figure 10** illustrates a joint for an articulation mechanism according to an aspect of the invention;
**Figure 11** illustrates a second view of the joint of Figure 10;
**Figure 12** illustrates a third view of the joint of Figure 10;
**Figure 13** illustrates an alternative articulation mechanism according to an aspect of the invention;
**Figure 14** illustrates a second view of the articulation mechanism of Figure 13;
**Figure 15** illustrates an articulation mechanism according to an aspect of the invention; and
**Figure 16** illustrates a second view of the articulation mechanism of Figure 15;

### Detailed Description of the Invention

Aspects and embodiments of the present invention will now be discussed with reference to the accompanying figures. Further aspects and embodiments will be apparent to those skilled in the art.

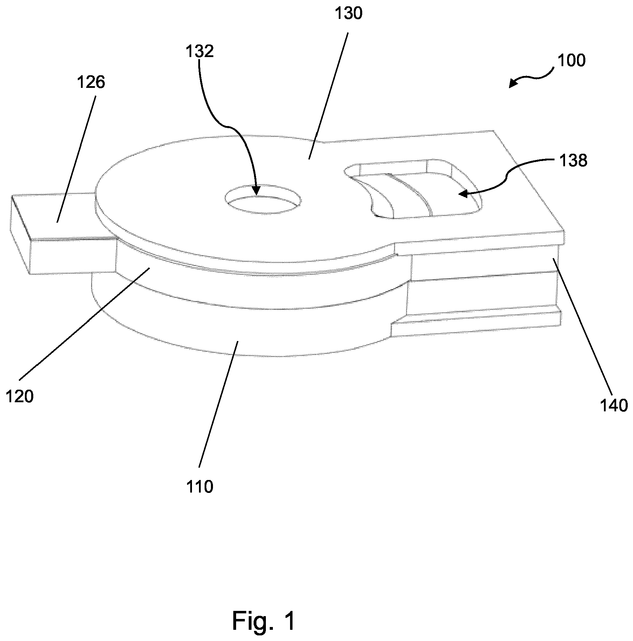

Figure 1 illustrates a planar joint 100 for an articulation mechanism according to an aspect of the invention. The joint of this arrangement comprises a first planar substrate 110, a second planar substrate 120, and a bracket 130 arranged to partially surround the first planar substrate 110 and the second planar substrate 120. The second planar substrate 120 of this arrangement is pivotably mounted with respect to the first planar substrate 110 and the bracket 130, and may thus rotate about a rotational axis passing through a pivot point of the joint 100, the rotational axis being perpendicular to the first planar substrate 110 and the second planar substrate 120.

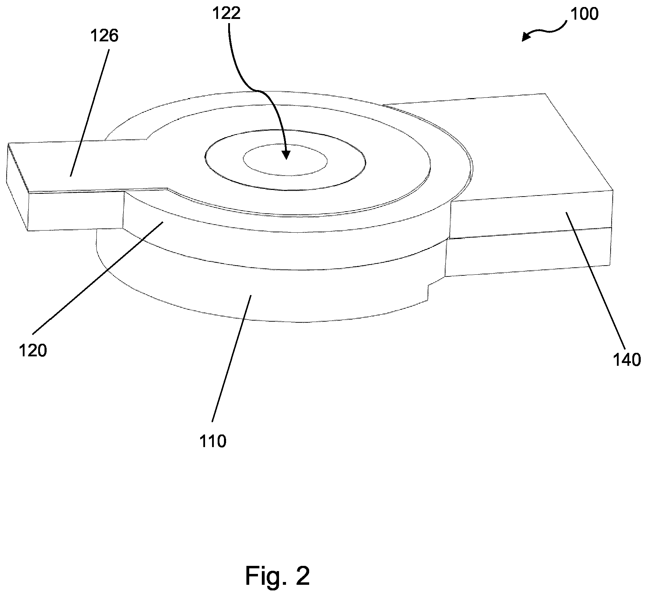

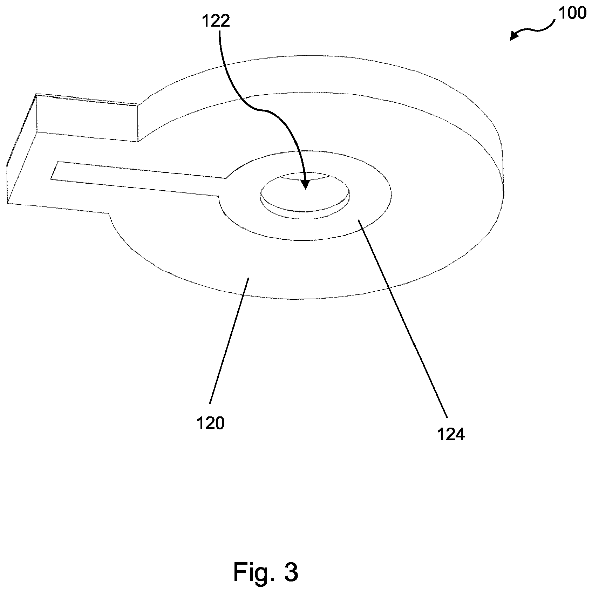

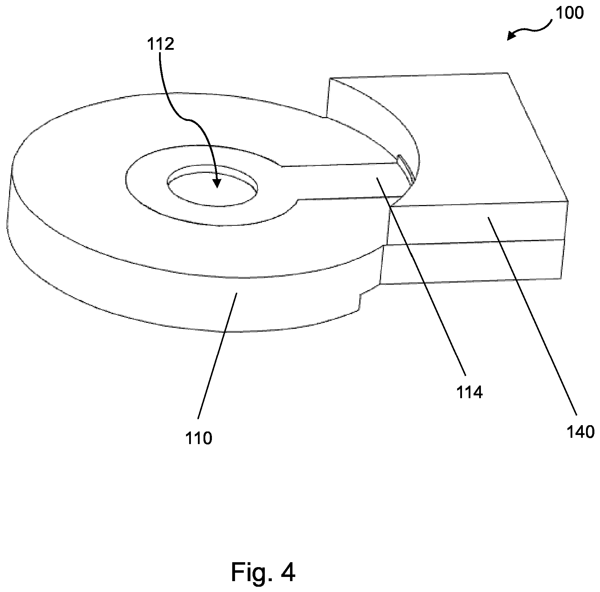

The bracket 130 of this arrangement includes a bore 132 located on the axis of rotation of the joint 100. The bore 132 may receive a pin or axle 160 about which the second planar substrate 120 of the joint 100 may pivot. The bracket 130 of this arrangement further includes a further hole 138, which may alter a breakdown voltage for the insulating parts of the joint 100. In other arrangements, bore 132 and or hole 138 may be omitted. The first planar substrate 110 may include a corresponding bore 112 (see Figure 4). The second planar substrate may also include a corresponding bore 122 (see Figures 2 and 3).

Figure 2 illustrates the joint 100 with the bracket 130 removed to show otherwise hidden features of the joint 100. As can be seen in Figure 2, the second planar substrate 120 of this arrangement has a conductive strip or coating (third conductive element 126) on the upper surface of the second planar substrate 120. The third conductive element 126 is therefore arranged to be in contact with the bracket 130 to form a part of a second electrical pathway through the joint 100. In other arrangements, the third conductive element 126 may not be present, for example when only a single electrical pathway is required through the joint.

As can be seen from Figure 3, the second planar substrate 120 of this arrangement further includes a conductive strip or coating (second conductive element 124) on a lower surface of the second planar substrate 120. The second conductive element 124 forms part of the first electrical pathway through the joint 100, and is arranged to contact a corresponding conductive coating (first conductive element 114) on an upper surface of the first planar substrate 110. The second conductive element 124 of this arrangement includes an annular portion surrounding the bore 122, and may therefore remain in contact with the first conductive element 114 at different rotation angles.

Figure 4 illustrates the first planar substrate 110 of the joint 100 of this arrangement to illustrate the otherwise hidden features of the joint 100. As illustrated in Figure 4, the first planar substrate 110 includes a conductive strip or coating (first conductive element 114) on an upper surface of the first planar substrate 100. The first conductive element 114 forms part of the first electrical pathway through the joint 100, and is arranged to contact the second conductive element 124. As with the second conductive element, the first conductive element 114 includes an annular portion surrounding the bore 112.

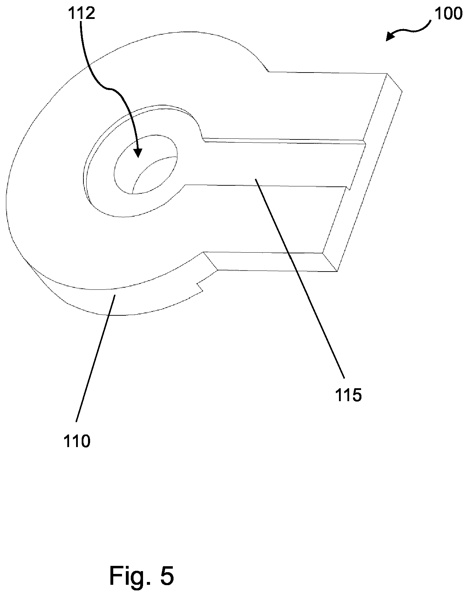

As can be seen in Figure 5, the first planar substrate 110 of this arrangement includes a trench 115 or recess on an upper surface of the first planar substrate 110. The trench 115 may receive the first conductive element 114, and thereby enable the first conductive element 114 to be located flush with the upper surface of the first planar substrate 110. The second conductive element 124 and third conductive element 126 may be arranged in corresponding trenches (not illustrated).

As can be seen in each of Figures 1 to 5, the first planar substrate 110 and the second planar substrate 120 of this arrangement include a circular portion surrounding the pivot point. The circular portion enables the first planar substrate 110 and the second planar substrate 120 to rotate more readily by ensuring that each has a constant radius at least part of the way about the pivot point. Other shapes for the planar substrates may also be used. The first planar substrate 110 and the second planar substrate 120 of this arrangement further include a rectangular portion (or tab portion) extending from the circular portion, such that each of the first planar substrate 110 and the second planar substrate 120 has a keyhole shape. The rectangular portion may be a different shape in other arrangements, or may be omitted. The rectangular portion may provide an increased surface for connecting an electrical connection to the conductive coatings 114. 124, 126 for delivering power to the joint 100 or receiving power from the joint 100.

As can be seen, for example, in Figure 4, the illustrated arrangement includes an abutment portion 140 which overlays the rectangular portion (or tab portion) of the first planar substrate 110. The abutment portion 140 is arranged to limit the rotational movement of the second planar substrate 120 with respect to the first planar substrate 110 so as to avoid a short circuit across the joint 100. The abutment portion 140 may be formed as part of the first planar substrate 110, or may be mounted with respect to the first planar substrate 110 and held in place by the bracket 130. In some arrangements, an abutment portion 140 may additionally or alternatively be arranged to overlay the rectangular portion of the second planar substrate 120.

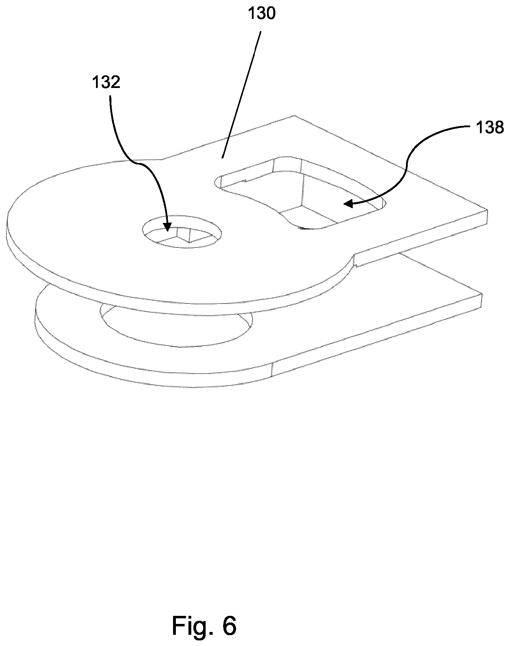

Figure 6 illustrates the bracket 130 of the joint 100 without the first planar substrate 110 or the second planar substrate 120. This more clearly illustrates the C-shaped nature of the bracket 130 of this arrangement, which enables the bracket to surround the pivot points of the first planar substrate 110 and the second planar substrate 120 to hold the joint 100 together.

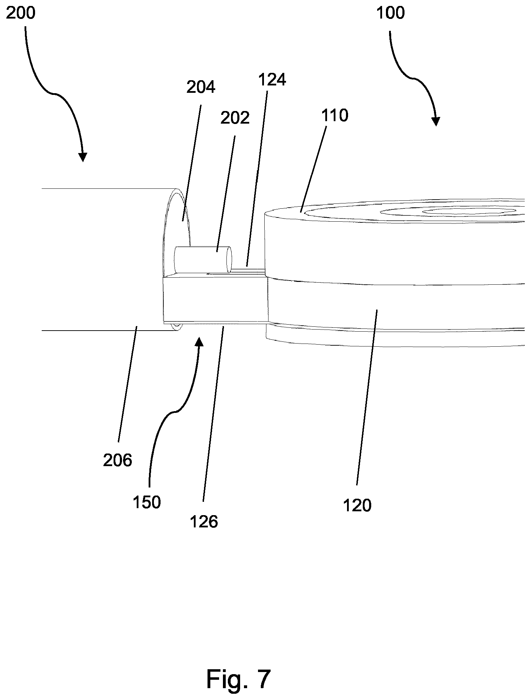

As illustrated exemplarily in Figure 7, the joint 100 may include a terminal 150 for connection to a cable, which in this arrangement is a coaxial cable 200. The coaxial cable 200 has an inner conductor 202 and an outer conductor 206 separated by a dielectric or insulating layer 204. In the illustrated arrangement, the inner conductor 202 is connected to the second conductive element 124 of the joint 100, and the outer conductor 206 is connected to the third conductive element 126 of the joint 100. A similar terminal may be provided to connect the bracket 130 and the first conductive element 122 to a coaxial cable. The terminal may therefore allow the joint 100 to be connected to an input power cable, for example connected to an electrosurgical generator. Alternatively, the terminal may allow the joint to be connected to an output power cable, for example connected to a further joint or to an electrosurgical instrument.

In other arrangements (not illustrated), the terminal may instead allow the joint 100 to be connected to a microstrip power cable. A microstrip power cable includes planar dielectric substrate having a first conductive coating or strip on an upper surface and a second conductive coating or strip on a lower surface.

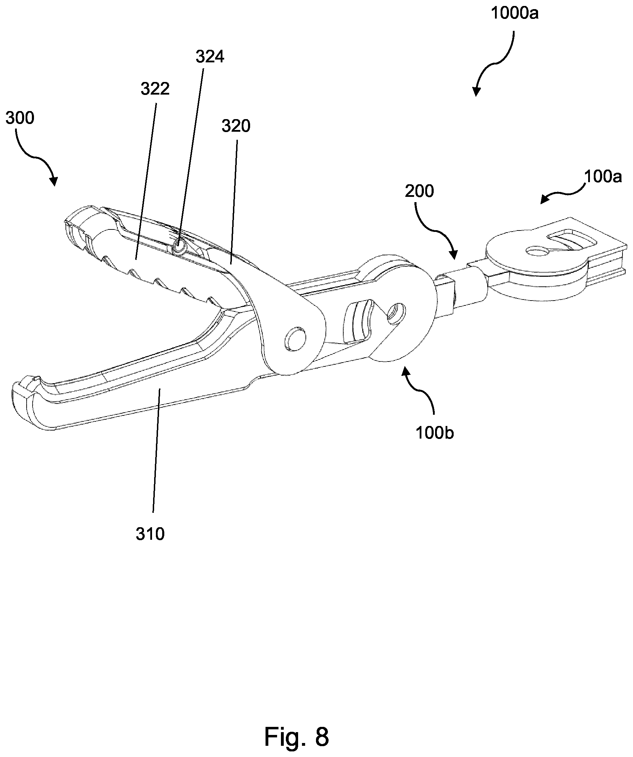

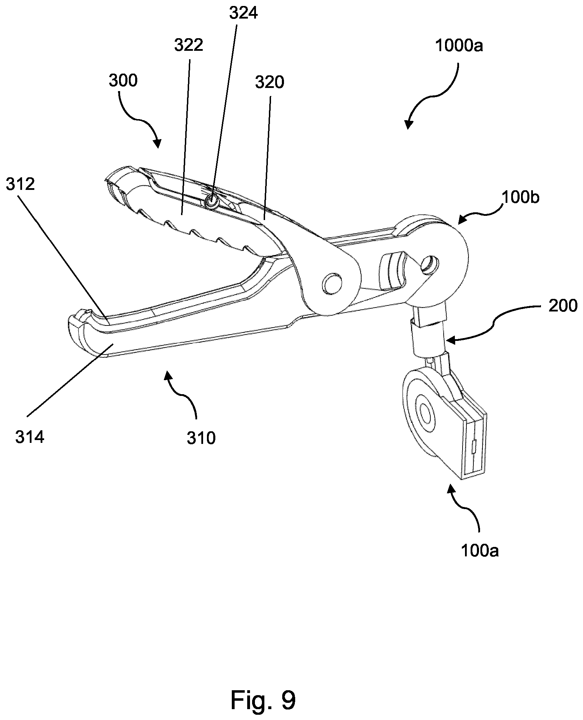

Figure 8 illustrates an articulation mechanism 1000a including two joints 100a, 100b connected by a coaxial cable 200. Other forms of connection, such as a microstrip connection may be used between the two joints 100a, 100b. Figure 8 shows the articulation mechanism 1000a with each of the two joints 100a, 100b in a straight (i.e. not pivoted) position. Figure 9 shows the articulation mechanism 1000a with each of the two joints 100a, 100b in a pivoted (i.e. rotated) position.

The articulation mechanism 1000a includes a first joint 100a and a second joint 100b mounted to a distal end of the first joint 100a via the cable 200. Therefore, as can be seen in Figure 9, rotating the first joint 100a moves the position of the second joint 100b. The first joint 100a and the second joint 100b of this arrangement have mutually perpendicular axes of rotation. In other arrangements, the axes of rotation may be offset (e.g. at different angles) but not perpendicular.

The illustrated articulation mechanism 1000a includes an electrosurgical instrument 300 having a fixed jaw 310 and a movable jaw 320. The movable jaw 320 is mounted to the fixed jaw 310 at a pivot point, the pivot point being distinct from the pivot point of the second joint 100b. In other examples, differing forms of electrosurgical instrument (for example an electrosurgical instrument that does not include a jaw) may be mounted or mountable to the articulation mechanism 1000a. For example, the electrosurgical instrument may be a probe arranged to deliver RF EM radiation and/or the microwave EM radiation separately or simultaneously from a distal end thereof. In an embodiment, a suitable probe may be as described in WO2011010086A1.

The fixed jaw 310 is integrally formed with the second joint 100b. This may reduce a size of the articulation mechanism 1000a by removing a requirement for a further connection between the second joint 100b and the electrosurgical instrument 300. Furthermore, since the second joint 100b and the electrosurgical instrument 300 are rigidly fixed together, undesirable flex between the second joint 100b and the electrosurgical instrument 300 may be reduced. In other arrangements, the second joint 100b may be connected to an electrosurgical instrument 300 via a further connection cable.

The electrosurgical instrument 300 may include a radiating structure, which in this example includes a first electrode 312 and a second electrode 314 arranged on the fixed jaw 310. The first electrode 312 is connected to the first electrical pathway of the articulation mechanism 1000a, and the second electrode 314 is connected to the second electrical pathway of the articulation mechanism 1000a. The first electrode 312 and second electrode 314 may be arranged to deliver electromagnetic energy (e.g. RF energy and/or microwave frequency energy) to biological tissue, for example to biological tissue held between the fixed jaw 310 and the moveable jaw 320.

In this example, the movable jaw 320 comprises a body made of a rigid material, e.g. metal, such as stainless steel. Mounted within the body is a back hinge plate 322. The back hinge plate 322 is pivotally connected to the distal end of the movable jaw, e.g. on a pin 324 that is mounted in the movable jaw 320. The back hinge plate 322 is arranged to pivot into a recess formed by the body of the movable jaw 320.

A resiliently deformable cushion element (not illustrated) is mounted on a back surface of the back hinge plate 322 to engage the inside surface of the movable jaw 320 when the back hinge plate 322 pivots into the recess. The resiliently deformable cushion element may be formed from silicone rubber or the like. The cushion element acts as a spring that is compressible under load as the pair of jaws is closed around a vessel or tissue bundle. On loading in this way it reduces the angle of inclination between the jaws as they are closed, thereby helping improve jaw alignment and parallelism earlier as the jaws are clamped together. This improves the evenness of pressure distribution across the vessel as it is clamped and improves stability, e.g. helps prevent a slippery vessel or tissue bundle from moving distally during jaw closure.

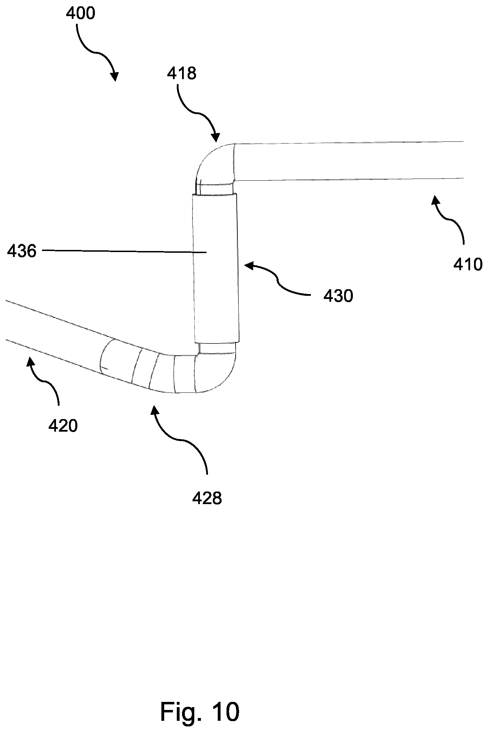

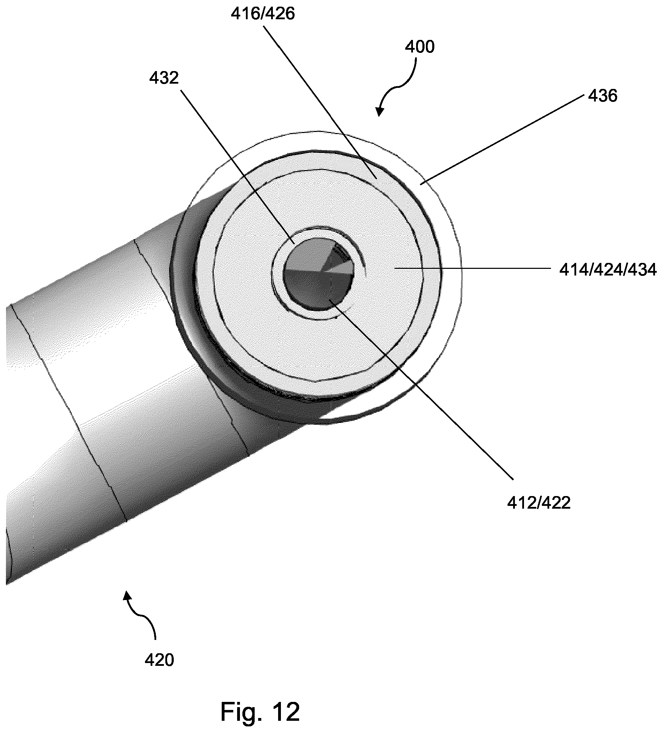

Figure 10 illustrates an alternative joint 400 that can be used in an articulation mechanism 1000. The illustrated joint 400 is a coaxial joint comprising a first coaxial cable 410, a second coaxial cable 420, and a rotatable connection 430. As seen on Fig. 12, each of the coaxial feed cables 410, 420 comprises a solid cylindrical inner conductor 412, 422, a tubular outer conductor 416, 426 that is coaxial with and surrounds the inner conductor 412, 422, and a dielectric material 414, 424 separating the inner 412, 422 and outer 416, 426 conductors.

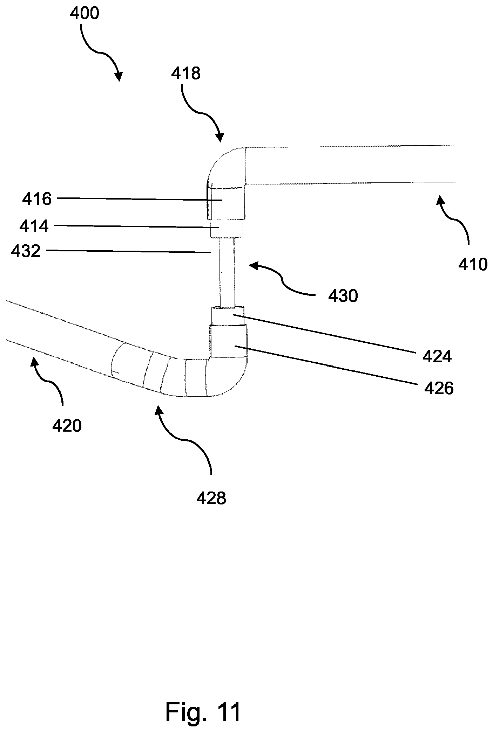

As illustrated in Fig. 11, a section of the dielectric material 414, 424 of each cable 410, 420 has been omitted or removed to leave a protruding distal end of the inner conductor 412, 422 that protrudes from the distal end of each cable 410, 420. A rotatable electric connection is formed between the inner conductors 412, 422 by a first conductive metal sleeve 432 provided over the protruding ends of the inner conductors 412, 422. The first conductive metal sleeve 432 is a metal tube with a diameter chosen so that the protruding ends of the inner conductors 412, 422 are rotatably received in the metal tube 432 and contact the metal tube 432 to form an electrical connection therebetween. This electrical connection forms part of the first electrical pathway through the joint.

Thus, the inner conductors 412, 422 are able to rotate relative to each other while an electrical connection is maintained there-between because of the rotatable connection provided by the first conductive metal sleeve 432.

In other arrangements, the conductive sleeve 432 may be fixedly connected to one of the inner conductors 412, 422. In other words, the conductive sleeve 432 may be rotatable with respect to only one of the inner conductors 412, 422.

As shown in Figure 10, a rotatable electrical connection is formed between the outer conductors 416, 426 of the coaxial cables 410, 420 via the provision of a second conductive metal sleeve 436 over the ends of the outer conductors 416, 426.

The second conductive metal sleeve 436 is a metal tube with a diameter chosen so that the ends of the outer conductors 416, 426 are rotatably received in the metal tube 436 and contact the metal tube 436 to form an electrical connection therebetween.

Thus, the outer conductors 416, 426 of the coaxial feed cables 410, 420 are able to rotate relative to each other while an electrical connection is maintained there-between because of the rotatable connection provided by the second conductive metal sleeve 436. This electrical connection forms part of the second electrical pathway through the joint.

In other arrangements, the conductive sleeve 436 may be fixedly connected to one of the outer conductors 416, 426. In other words, the conductive sleeve 436 may be rotatable with respect to only one of the outer conductors 416, 426. In still further arrangements (such as when only a single electrical pathway is required) the conductive sleeve 436 may be omitted.

Thus, the combination of the first and second conductive metal sleeves 432, 436 provides a rotatable connection 430 between the coaxial cables 410, 420 that allows the coaxial feed cables 410, 420 to be rotated relative to each other, while maintaining an electrical connection between the coaxial cables 410, 420.

Electromagnetic energy (e.g. RF energy and/or microwave frequency energy) can be transmitted from one coaxial cable 410 to the other coaxial cable 420 through the rotatable connection 430 because of the rotatable electrical connections provided by the first and second conductive metal sleeves 432, 436.

The first and second conductive metal sleeves 432, 436 form a coaxial transmission line for conveying the RF energy and/or microwave energy with air as the dielectric material. In other arrangements, a dielectric filler material 434 may be provided between the first and second conductive metal sleeves 432, 436, as illustrated in Figure 12, which shows a cross-section through the joint 400.

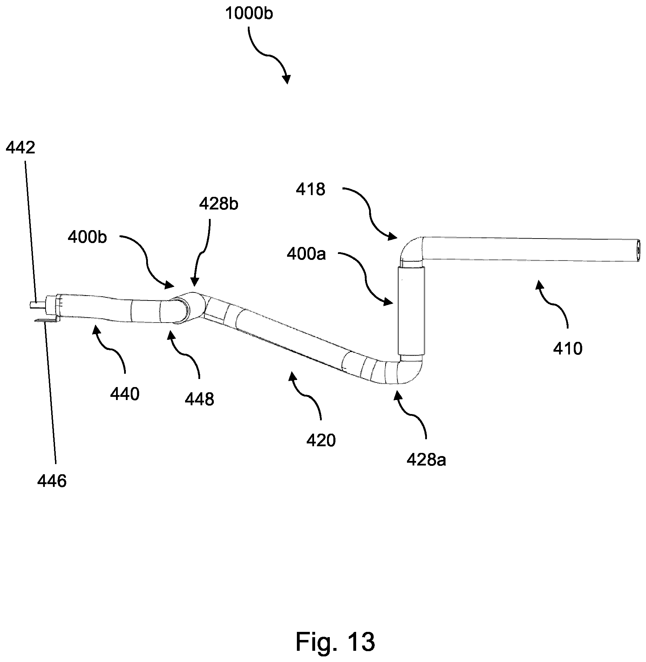

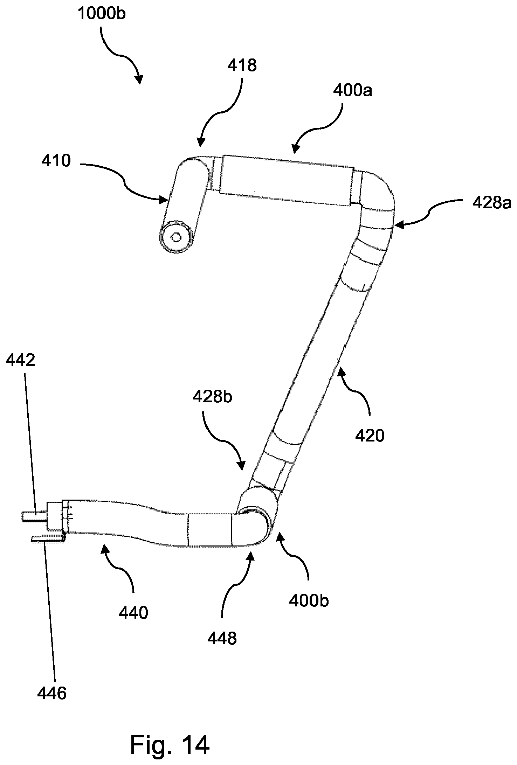

Figures 13 and 14 illustrate an articulation mechanism 1000b including two joints 400a, 400b connected by a coaxial cable 420. Figure 13 shows the articulation mechanism 1000b with each of the two joints 400a, 400b in a straight (i.e. not pivoted) position. Figure 14 shows the articulation mechanism 1000b with each of the two joints 400a, 400b in a pivoted (i.e. rotated) position.

The illustrated arrangement includes a connector at a distal end of the articulation mechanism for connection to an electrosurgical instrument. The connector includes a first terminal 442 for connecting the electrosurgical instrument to the first electrical pathway through the articulation mechanism 1000b, and a second terminal 446 for connecting the electrosurgical instrument to the second electrical pathway through the articulation mechanism 1000b. A similar connector may be provided at a proximal end of the articulation mechanism for connection to a power cable to receive power from an electrosurgical generator. A terminal of this type may also be utilised in combination with the articulation mechanism 1000a illustrated in Figures 8 and 9.

The rotation axes of the first coaxial joint 400a and second coaxial joint 400b may be aligned relative to one another through the provision of one or more bends 418, 428a, 428b, 448 in the coaxial cables 410, 420, 440. The rotation axes of the first coaxial joint 400a and second coaxial joint 400b are parallel with an axial direction of the joint, and therefore a bend may be required to offset the rotation axes. In the illustrated arrangement, the bends 418, 428a, 428b, 448 position the rotation axes of the joints to be perpendicular to one another, and to be perpendicular to the axial direction of the electrical input and output of the articulation mechanism 100b. Other configurations are also possible, as may be required by the geometry of the attached instrument, and/or other components of the electrosurgical device.

The two illustrated articulation mechanisms 1000a, 1000b have each included two joints of the same type. However, in other examples, the articulation mechanism may instead include more than one type of joint. For example, the articulation mechanism may include a planar joint 100 and a coaxial joint 400. The number of joints is also not limited to two. In some articulation mechanisms, more than two joints may be included to provide the desired steering action.

The articulation mechanism 1000a, 1000b may form the wrist of a robotic arm configured to control positioning of an electrosurgical instrument. Alternatively, the articulation mechanism 1000a, 1000b may be mountable within the wrist of a robotic arm. In this way, the articulation mechanism can convey electromagnetic energy (e.g. microwave energy or RF energy) through the wrist of a robotic arm system. For example, the articulation mechanism 1000a, 1000b may be configured to meet a particular outer dimension and/or footprint and/or minimum bending radius to be compatible with the robotic arm system.

To protect the articulation mechanism 1000a, 1000b, and/or the components from physical damage and/or from surrounding conditions such moisture or fluid ingress, the articulation mechanism 1000a, 1000b may comprise an outer housing. For example, the articulation mechanism 1000a, 1000b may be housed within a flexible sleeve or sheath. In other arrangements, each joint of the articulation mechanism may be housed within a separate housing.

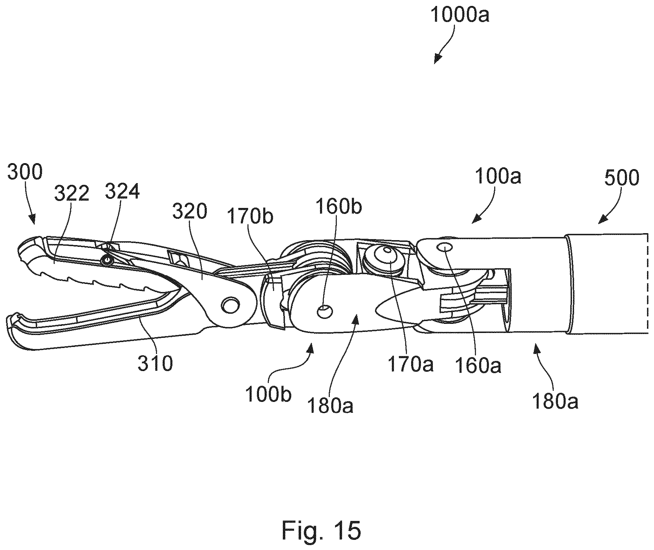

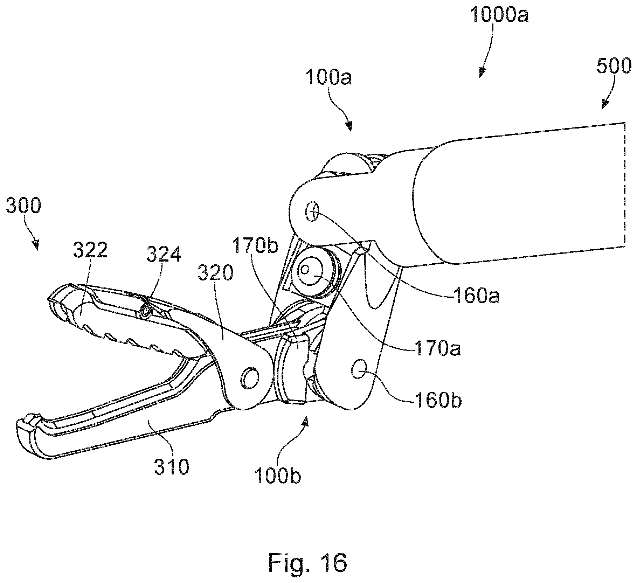

Figures 15 and 16 illustrate an assembled articulation mechanism 1000a to more clearly illustrate how the articulation mechanism may be arranged within a system. Figure 15 shows the articulation mechanism 1000a with each of the two joints 100a, 100b in a straight (i.e. not pivoted) position. Figure 16 shows the articulation mechanism 1000a with each of the two joints 100a, 100b in a pivoted (i.e. rotated) position.

The articulation mechanism may be mounted at a distal end of an instrument shaft 500. The instrument shaft 500 may, for example, contain control wires or rods for articulating the joints 100a, 100b and/or the jaws 310, 320 of the electrosurgical instrument 300. The instrument shaft 500 may also contain one or more power cables for providing electromagnetic (EM) energy (e.g. radiofrequency RF EM energy and/or microwave frequency EM energy) to the articulation mechanism 1000a for conveyance to the electrosurgical instrument 300. The instrument shaft may contain additional components, such as channels for supplying or extracting gas or fluid (e.g. saline) and/or further control mechanisms (for example for an element such as a retractable blade).

Figures 15 and 16 also illustrate an example of axles 160a, 160b that are arranged to pass through the pivot point of the joints 100a, 100b. These axles 160a, 160b may further connect the joints 100a, 100b to a joint mounting bracket 180a, 180b within which the joint 100a, 100b may be mounted. These joint mounting brackets 180a, 180b may be electrically isolated from the respective electrical pathways of the joints 100a, 100b. The joint mounting brackets 180a, 180b may be configured to provide a level of physical protection for the joints 100a, 100b, for example against impact or intrusion.

Figures 15 and 16 further indicate an exemplary articulating actuators (or means) 170a, 170b for the joints. In this example, the joints or actuators include a peg or pulley 170a, 170b which may be acted upon by a control wire (not illustrated) to articulate the joints 100a, 100b. In other embodiments, different control mechanisms or actuators may be used. These may include control mechanisms (such as a servo or motor) mounted to the joint 100a, 100b, and/or other means of conveying an articulating force via a force transfer element from a proximal end of the instrument shaft 500.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

## Claims

1. A joint (100) for an articulation mechanism for use in electrosurgery, the joint comprising
a first electrically insulating planar substrate (110) having a first surface and a second surface opposite the first surface,
a second electrically insulating planar substrate (120) having a first surface and a second surface opposite the first surface, the second planar substrate being mounted to be rotatable about a pivot point with respect to the first planar substrate such that the first surface of the first planar substrate is in contact with the second surface of the second planar substrate;
a first conductive element (114) arranged on the first surface of the first planar substrate; a second conductive element (124) arranged on the second surface of the second planar substrate;
such that the first and second conductive elements form a first electrical pathway through the joint; and
**characterised by**:
a third conductive element (126) arranged on the first surface of the second planar substrate;
such that the third conductive element forms part of a second electrical pathway through the joint, the second electrical pathway being separate from the first electrical pathway.

2. A joint according to claim 1, wherein the first conductive element and the second conductive element are in direct electrical contact, and wherein the first conductive element and the second conductive element are configured to slide against one another to maintain direct electrical contact when the second planar substrate is rotated with respect to the first planar substrate; and
optionally, wherein at least one of the first conductive element and the second conductive element is a conductive coating applied to the respective surface of the respective planar substrate.

3. A joint according to claim 1, further comprising:
an electrically conductive bracket (130) mounted in contact with the first surface of the second plate and being configured to hold the first and second plates together; wherein
the third conductive element and the bracket form the second electrical pathway through the joint; and
optionally, wherein at least one of the bracket and the first planar substrate is integrally formed with a part of an electrosurgical instrument; and
optionally, wherein the electrosurgical instrument comprises a radiating structure for delivering electromagnetic energy received from the first and second electrical pathways through the joint to biological tissue.

4. A joint according to any preceding claim, wherein the each of the first electrical pathway and the second electrical pathway are arranged to convey electromagnetic energy from an electrosurgical generator through the joint, and wherein the joint comprises a connector for connecting the first and second electrical pathways to a power cable connected to the electrosurgical generator.

5. A joint according to claim 4, wherein the connector is configured:
to connect to a coaxial cable as the power cable, the coaxial cable including an inner conductor and an outer conductor separated by a dielectric layer, and wherein the connector includes a first terminal for connecting the inner connector to the first electrical pathway and a second terminal for connecting the outer conductor to the second electrical pathway; or
to connect to a microstrip as the power cable, the microstrip including first and second conductors separated by a planar dielectric layer, and wherein the connector includes a first terminal for connecting the first conductor to the first electrical pathway and a second terminal for connecting the second conductor to the second electrical pathway.

6. A joint according to any preceding claim, wherein at least one of the first planar substrate and the second planar substrate comprises a circular portion about the pivot point; and
optionally, wherein at least one of the following applies:
a) the respective planar substrate comprises a circular bore at a central position of the circular portion;
b) the conductive element of the respective planar substrate forms a ring around the circular bore.

7. A joint according to claim 5 or 6, wherein at least one of the first planar substrate and the second planar substrate has a keyhole shape, comprising a circular portion about the pivot point and a rectangular portion (115) extending from the circular portion.

8. An articulation mechanism (1000a) for steering an electrosurgical instrument, the articulation mechanism having a proximal end and a distal end, the articulation mechanism comprising:
a first terminal located at the proximal end of the articulation mechanism for receiving electromagnetic energy from an electrosurgical generator;
a second terminal located at the distal end of the articulation mechanism for delivering the electromagnetic energy received from the electrosurgical generator to the electrosurgical instrument;
a first rotating joint (100a) having a first axis of rotation; and
**characterised by**:
a second rotating joint (100b) arranged distally of the first rotating joint and having a second axis of rotation different from the first axis of rotation; wherein
the first and second rotating joints each comprise:
a first joint element having a first conductive element on a surface thereof;
a second joint element having a second conductive element on a surface thereof; the second joint element being rotatably mounted with respect to the first joint element such that the surface of the first joint element having the conductive element is in contact with the surface of the second joint element having the conductive element such that the first and second conductive elements form a first electrical pathway through the joint; and wherein
one of the first and second joint elements of each of the first and second joints includes a third conductive element on a second surface thereof, the third conductive element forming part of a second electrical pathway through each joint, the second electrical pathway being separate from the first electrical pathway.

9. An articulation mechanism according to claim 8, wherein the first and second axes of rotation are perpendicular to one another.

10. An articulation mechanism according to any of claims 8 to 9, wherein the first rotating joint and the second rotating joint are connected via a connection comprising a coaxial cable.

11. An articulation mechanism according to claim 10, wherein
at least one of the first rotating joint and the second rotating joint is a coaxial rotating joint comprising:
a first coaxial cable and a second coaxial cable, each coaxial cable comprising an inner conductor and an outer conductor separated by a dielectric layer, wherein the first coaxial cable forms the first joint element;
a first conductive sleeve forming part of the first electrical pathway, and arranged to form a rotatable electrical connection between the respective inner conductors, wherein the first conductive sleeve forms the second joint element; and
a second conductive sleeve forming part of the second electrical pathway, and arranged to form a rotatable electrical connection between the respective outer conductors; and
optionally, wherein the coaxial rotating joint further comprises an electrically insulating sleeve arranged between the first conductive sleeve and the second conductive sleeve.

12. An articulation mechanism according to claim 10 or 11, wherein each of the first rotating joint and the second rotating joint is a coaxial rotating joint, and wherein the connection between the first rotating joint and the second rotating joint comprises one or more bends (418, 428a, 428b, 448) to introduce an offset of the second axis of rotation compared to the first axis of rotation such that the second axis of rotation is different from the first axis of rotation.

13. An articulation mechanism according to claim 8 or 9, wherein the first rotating joint and the second rotating joint are connected via a connection comprising a microstrip, the microstrip comprising first and second conductors separated by a planar dielectric layer.

14. An articulation mechanism according to any of claims 9 to 13, wherein
at least one of the first rotating joint and the second rotating joint is a joint according to any one of claims 1 to 8, with the first joint element being the first planar substrate and the second joint element being the second planar substrate.

15. An articulation mechanism according to any of claims 9 to 14, further comprising one of:
a connector for mounting an electrosurgical instrument to the articulation mechanism; or
an electrosurgical instrument integrally formed with the first joint element or the second joint element of the second rotating joint.

16. An electrosurgical device comprising
an articulation mechanism according to any of claims 8 to 15; and
an electrosurgical instrument mounted at the distal end of the articulation mechanism.

## Patentansprüche

1. Gelenk (100) für einen Gelenkmechanismus zur elektrochirurgischen Verwendung, wobei das Gelenk Folgendes umfasst:
ein erstes elektrisch isolierendes planares Substrat (110) mit einer ersten Fläche und einer zweiten Fläche entgegengesetzt zu der ersten Fläche,
ein zweites elektrisch isolierendes planares Substrat (120) mit einer ersten Fläche und einer zweiten Fläche entgegengesetzt zu der ersten Fläche, wobei das zweite planare Substrat angebracht ist, um derart um einen Schwenkpunkt in Bezug auf das erste planare Substrat drehbar zu sein, dass die erste Fläche des ersten planaren Substrats mit der zweiten Fläche des zweiten planaren Substrats in Kontakt ist;
ein erstes leitfähiges Element (114), das auf der ersten Fläche des ersten planaren Substrats angeordnet ist;
ein zweites leitfähiges Element (124), das auf der zweiten Fläche des zweiten planaren Substrats angeordnet ist;
sodass das erste und das zweite leitfähige Element einen ersten elektrischen Pfad durch das Gelenk ausbilden; und
**gekennzeichnet durch**:
ein drittes leitfähiges Element (126), das auf der ersten Fläche des zweiten planaren Substrats angeordnet ist;
sodass das dritte leitfähige Element einen Teil eines zweiten elektrischen Pfads durch das Gelenk ausbildet, wobei der zweite elektrische Pfad von dem ersten elektrischen Pfad getrennt ist.

2. Gelenk nach Anspruch 1, wobei das erste leitfähige Element und das zweite leitfähige Element in direktem elektrischem Kontakt sind und wobei das erste leitfähige Element und das zweite leitfähige Element dazu ausgelegt sind, gegeneinander zu gleiten, um einen direkten elektrischen Kontakt aufrechtzuerhalten, wenn das zweite planare Substrat in Bezug auf das erste planare Substrat gedreht wird; und
wobei gegebenenfalls zumindest eines aus dem ersten leitfähigen Element und dem zweiten leitfähigen Element eine leitfähige Beschichtung ist, welche auf die entsprechende Fläche des entsprechenden planaren Substrats aufgebracht ist.

3. Gelenk nach Anspruch 1, ferner umfassend:
eine elektrisch leitfähige Klammer (130), die im Kontakt mit der ersten Fläche der zweiten Platte montiert und dazu ausgelegt ist, die erste und die zweite Platte zusammenzuhalten; wobei
das dritte leitfähige Element und die Klammer den zweiten elektrischen Pfad durch das Gelenk ausbilden; und
wobei gegebenenfalls zumindest eines aus der Klammer und dem ersten planaren Substrat einstückig mit einem Teil eines elektrochirurgischen Instruments ausgebildet ist; und
wobei das elektrochirurgische Instrument gegebenenfalls eine Abstrahlstruktur zum Abgeben von elektromagnetischer Energie, die von dem ersten und dem zweiten elektrischen Pfad durch das Gelenk empfangen wurde, an biologisches Gewebe umfasst.

4. Gelenk nach einem der vorangegangenen Ansprüche, wobei jeder aus dem ersten elektrischen Pfad und dem zweiten elektrischen Pfad angeordnet ist, um elektromagnetische Energie von einem elektrochirurgischen Generator durch das Gelenk hindurch zu übertragen und wobei das Gelenk einen Verbinder zum Verbinden des ersten und des zweiten elektrischen Pfads mit einem Stromkabel, das mit dem elektrochirurgischen Generator verbunden ist, umfasst.

5. Gelenk nach Anspruch 4, wobei der Verbinder für Folgendes ausgelegt ist:
Verbinden mit einem Koaxialkabel als das Stromkabel, wobei das Koaxialkabel einen Innenleiter und einen Außenleiter umfasst, die durch eine dielektrische Schicht getrennt sind, und wobei der Verbinder einen ersten Anschluss zum Verbinden des Innenleiters mit dem ersten elektrischen Pfad und einen zweiten Anschluss zum Verbinden des Außenleiters mit dem zweiten elektrischen Pfad umfasst; oder
Verbinden eines Mikrostreifens als das Stromkabel, wobei der Mikrostreifen einen ersten und einen zweiten Leiter, die durch eine planare dielektrische Schicht getrennt sind, umfasst, und wobei der Verbinder einen ersten Anschluss zum Verbinden des ersten Leiters mit dem ersten elektrischen Pfad und einen zweiten Anschluss zum Verbinden des zweiten Leiters mit dem zweiten elektrischen Pfad umfasst.

6. Gelenk nach einem der vorangegangenen Ansprüche, wobei zumindest eines aus dem ersten planaren Substrat und dem zweiten planaren Substrat einen kreisförmigen Abschnitt um den Schwenkpunkt umfasst; und
wobei gegebenenfalls zumindest eines aus folgenden zutrifft:
a) das entsprechende planare Substrat umfasst eine kreisförmige Bohrung an einer zentralen Stelle des kreisförmigen Abschnitts;
b) das leitfähige Element des entsprechenden planaren Substrats bildet einen Ring um die kreisförmige Bohrung aus.

7. Gelenk nach Anspruch 5 oder 6, wobei zumindest eines aus dem ersten planaren Substrat und dem zweiten planaren Substrat eine Schlüssellochform aufweist, umfassend einen kreisförmigen Abschnitt um den Schwenkpunkt und einen rechteckigen Abschnitt (115), der sich vom kreisförmigen Abschnitt weg erstreckt.

8. Gelenkmechanismus (1000a) zum Lenken eines elektrochirurgischen Instruments, wobei der Gelenkmechanismus ein proximales Ende und ein distales Ende aufweist, wobei der Gelenkmechanismus Folgendes umfasst:
einen ersten Anschluss, der am proximalen Ende des Gelenkmechanismus angeordnet ist, zum Empfangen von elektromagnetischer Energie von einem elektrochirurgischen Generator;
einen zweiten Anschluss, der am distalen Ende des Gelenkmechanismus angeordnet ist, zum Abgeben der vom elektrochirurgischen Generator empfangenen elektromagnetischen Energie an das elektrochirurgische Instrument;
ein erstes Drehgelenk (100a) mit einer ersten Drehachse; und
**gekennzeichnet durch**:
ein zweites Drehgelenk (100b), das distal von dem ersten Drehgelenk angeordnet ist und eine zweite Drehachse aufweist, die sich von der ersten Drehachse unterscheidet; wobei
das erste und das zweite Drehgelenk jeweils Folgendes umfassen:
ein erstes Gelenkelement mit einem ersten leitfähigen Element auf einer Fläche desselben;
ein zweites Gelenkelement mit einem zweiten leitfähigen Element auf einer Fläche desselben, wobei das zweite Gelenkelement derart drehbar in Bezug auf das erste Gelenkelement montiert ist, dass die Fläche des ersten Gelenkelements, welches das leitfähige Element aufweist, mit der Fläche des zweiten Gelenkelements, welches das leitfähige Element aufweist, in Kontakt ist, sodass das erste und das zweite leitfähige Element einen ersten elektrischen Pfad durch das Gelenk ausbilden; und wobei
eines aus dem ersten und dem zweiten Gelenkelement von jedem aus dem ersten und dem zweiten Gelenk ein drittes leitfähiges Element auf einer zweiten Fläche desselben umfasst, wobei das dritte leitfähige Element einen Teil eines zweiten elektrischen Pfads durch jedes Gelenk ausbildet, wobei der zweite elektrische Pfad von dem ersten elektrischen Pfad getrennt ist.

9. Gelenkmechanismus nach Anspruch 8, wobei die erste und die zweite Drehachse senkrecht aufeinander sind.

10. Gelenkmechanismus nach einem der Ansprüche 8 bis 9, wobei das erste Drehgelenk und das zweite Drehgelenk über eine Verbindung, die ein Koaxialkabel umfasst, verbunden sind.

11. Gelenkmechanismus nach Anspruch 10, wobei
zumindest eines aus dem ersten Drehgelenk und dem zweiten Drehgelenk ein koaxiales Drehgelenk ist, welches Folgendes umfasst:
ein erstes Koaxialkabel und ein zweites Koaxialkabel, wobei jedes Koaxialkabel einen Innenleiter und einen Außenleiter umfasst, die durch eine dielektrische Schicht getrennt sind, wobei das erste Koaxialkabel das erste Gelenkelement ausbildet;
eine erste leitfähige Hülse einen Teil des ersten elektrischen Pfads ausbildet und angeordnet ist, um eine drehbare elektrische Verbindung zwischen den entsprechenden Innenleitern auszubilden, wobei die erste leitfähige Hülse das zweite Gelenkelement ausbildet; und
eine zweite leitfähige Hülse einen Teil des zweiten elektrischen Pfads ausbildet und angeordnet ist, um eine drehbare elektrische Verbindung zwischen den entsprechenden Außenleitern auszubilden; und
wobei das koaxiale Drehgelenk gegebenenfalls ferner eine elektrisch isolierende Hülse umfasst, die zwischen der ersten leitfähigen Hülse und der zweiten leitfähigen Hülse angeordnet ist.

12. Gelenkmechanismus nach Anspruch 10 oder 11, wobei jedes aus dem ersten Drehgelenk und dem zweiten Drehgelenk ein koaxiales Drehgelenk ist und wobei die Verbindung zwischen dem ersten Drehgelenk und dem zweiten Drehgelenk eine oder mehrere Biegungen (418, 428a, 428b, 448) zum Einführen eines Versatzes der zweiten Drehachse verglichen mit der ersten Drehachse umfasst, sodass die zweite Drehachse sich von der ersten Drehachse unterscheidet.

13. Gelenkmechanismus nach Anspruch 8 oder 9, wobei das erste Drehgelenk und das zweite Drehgelenk über eine Verbindung, die einen Mikrostreifen umfasst, verbunden sind, wobei der Mikrostreifen einen ersten und einen zweiten Leiter umfasst, die durch eine planare dielektrische Schicht getrennt sind.

14. Gelenkmechanismus nach einem der Ansprüche 9 bis 13, wobei
zumindest eines aus dem ersten Drehgelenk und dem zweiten Drehgelenk ein Gelenk gemäß einem der Ansprüche 1 bis 8 ist, wobei das erste Gelenkelement das erste planare Substrat ist und das zweite Gelenkelement das zweite planare Substrat ist.

15. Gelenkmechanismus nach einem der Ansprüche 9 bis 14, ferner umfassend eines aus Folgendem:
einen Verbinder zum Montieren eines elektrochirurgischen Instruments an dem Gelenkmechanismus; oder
ein elektrochirurgisches Instrument, das einstückig mit dem ersten Gelenkelement oder dem zweiten Gelenkelement des zweiten Drehgelenks ausgebildet ist.

16. Elektrochirurgische Vorrichtung, umfassend:
einen Gelenkmechanismus nach einem der Ansprüche 8 bis 15; und
ein elektrochirurgisches Instrument, das an dem distalen Ende des Gelenkmechanismus montiert ist.

## Revendications

1. Joint (100) pour un mécanisme d'articulation destiné à être utilisé en électrochirurgie, le joint comprenant
un premier substrat plan électriquement isolant (110) présentant une première surface et une seconde surface opposée à la première surface,
un second substrat plan électriquement isolant (120) présentant une première surface et une seconde surface opposée à la première surface, le second substrat plan étant monté pour pouvoir tourner autour d'un point de pivotement par rapport au premier substrat plan de sorte que la première surface du premier substrat plan soit en contact avec la seconde surface du second substrat plan ;
un premier élément conducteur (114) agencé sur la première surface du premier substrat plan ;
un deuxième élément conducteur (124) agencé sur la seconde surface du second substrat plan ;
de sorte que les premier et deuxième éléments conducteurs forment un premier trajet électrique à travers le joint ; et
**caractérisé par** :
un troisième élément conducteur (126) agencé sur la première surface du second substrat plan ;
de sorte que le troisième élément conducteur forme une partie d'un second trajet électrique à travers le joint, le second trajet électrique étant séparé du premier trajet électrique.

2. Joint selon la revendication 1, dans lequel le premier élément conducteur et le deuxième élément conducteur sont en contact électrique direct, et dans lequel le premier élément conducteur et le deuxième élément conducteur sont configurés pour coulisser l'un contre l'autre pour maintenir un contact électrique direct lorsque le second substrat plan est tourné par rapport au premier substrat plan ; et
facultativement, dans lequel le premier élément conducteur et/ou le deuxième élément conducteur est/sont un revêtement conducteur appliqué sur la surface respective du substrat plan respectif.

3. Joint selon la revendication 1, comprenant en outre :
un support électriquement conducteur (130) monté en contact avec la première surface de la seconde plaque et configuré pour maintenir les première et seconde plaques ensemble ; dans lequel
le troisième élément conducteur et le support forment le second trajet électrique à travers le joint ; et
facultativement, dans lequel le support et/ou le premier substrat plan est/sont formé(s) d'un seul tenant avec une partie d'un instrument électrochirurgical ; et
facultativement, dans lequel l'instrument électrochirurgical comprend une structure rayonnante permettant de délivrer une énergie électromagnétique reçue à partir des premier et second trajets électriques à travers le joint à un tissu biologique.

4. Joint selon une quelconque revendication précédente, dans lequel chacun du premier trajet électrique et du second trajet électrique est agencé pour transporter une énergie électromagnétique à partir d'un générateur électrochirurgical à travers le joint, et dans lequel le joint comprend un connecteur permettant de connecter les premier et second trajets électriques à un câble d'alimentation connecté au générateur électrochirurgical.

5. Joint selon la revendication 4, dans lequel le connecteur est configuré :
pour se connecter à un câble coaxial en tant que câble d'alimentation, le câble coaxial comprenant un conducteur interne et un conducteur externe séparés par une couche diélectrique, et dans lequel le connecteur comprend une première borne pour connecter le connecteur interne au premier trajet électrique et une seconde borne pour connecter le conducteur externe au second trajet électrique ; ou
pour se connecter à une microbande en tant que câble d'alimentation, la microbande comprenant des premier et second conducteurs séparés par une couche diélectrique plane, et dans lequel le connecteur comprend une première borne pour connecter le premier conducteur au premier trajet électrique et une seconde borne pour connecter le second conducteur au second trajet électrique.

6. Joint selon une quelconque revendication précédents, dans lequel au moins l'un du premier substrat plan et du second substrat plan comprend une partie circulaire autour du point de pivotement ; et
facultativement, dans lequel au moins l'un des points suivants s'applique :
a) le substrat plan respectif comprend un alésage circulaire au niveau d'une position centrale de la partie circulaire ;
b) l'élément conducteur du substrat plan respectif forme un anneau autour de l'alésage circulaire.

7. Joint selon la revendication 5 ou 6, dans lequel le premier substrat plan et/ou le second substrat plan présente(nt) une forme de trou de serrure, comprenant une partie circulaire autour du point de pivotement et une partie rectangulaire (115) s'étendant à partir de la partie circulaire.

8. Mécanisme d'articulation (1000a) permettant de diriger un instrument électrochirurgical, le mécanisme d'articulation présentant une extrémité proximale et une extrémité distale, le mécanisme d'articulation comprenant :
une première borne située au niveau de l'extrémité proximale du mécanisme d'articulation permettant de recevoir une énergie électromagnétique d'un générateur électrochirurgical ;
une seconde borne située au niveau de l'extrémité distale du mécanisme d'articulation permettant de délivrer l'énergie électromagnétique reçue à partir du générateur électrochirurgical à l'instrument électrochirurgical ;
un premier joint tournant (100a) présentant un premier axe de rotation ; et
**caractérisé par** :
un second joint tournant (100b) agencé de manière distale par rapport au premier joint tournant et présentant un second axe de rotation différent du premier axe de rotation ; dans lequel
les premier et second joints tournants comprennent chacun :
un premier élément de joint présentant un premier élément conducteur sur une surface de celui-ci ;
un second élément de joint présentant un deuxième élément conducteur sur une surface de celui-ci ; le second élément de joint étant monté en rotation par rapport au premier élément de joint de sorte que la surface du premier élément de joint présentant l'élément conducteur soit en contact avec la surface du second élément de joint présentant l'élément conducteur de sorte que les premier et deuxième éléments conducteurs forment un premier trajet électrique à travers le joint ; et dans lequel
l'un des premier et second éléments de joint de chacun des premier et second joints comprend un troisième élément conducteur sur une seconde surface de celui-ci, le troisième élément conducteur faisant partie d'un second trajet électrique à travers chaque joint, le second trajet électrique étant séparé du premier trajet électrique.

9. Mécanisme d'articulation selon la revendication 8, dans lequel les premier et second axes de rotation sont perpendiculaires l'un à l'autre.

10. Mécanisme d'articulation selon l'une quelconque des revendications 8 à 9, dans lequel le premier joint tournant et le second joint tournant sont connectés via une connexion comprenant un câble coaxial.

11. Mécanisme d'articulation selon la revendication 10, dans lequel :
le premier joint tournant et/ou le second joint tournant est/sont un joint tournant coaxial comprenant :
un premier câble coaxial et un second câble coaxial, chaque câble coaxial comprenant un conducteur interne et un conducteur externe séparés par une couche diélectrique, dans lequel le premier câble coaxial forme le premier élément de joint ;
un premier manchon conducteur faisant partie du premier trajet électrique, et agencé pour former une connexion électrique tournante entre les conducteurs internes respectifs, dans lequel le premier manchon conducteur forme le second élément de joint ; et
un second manchon conducteur faisant partie du second trajet électrique, et agencé pour former une connexion électrique tournante entre les conducteurs externes respectifs ; et
facultativement, dans lequel le joint tournant coaxial comprend en outre un manchon électriquement isolant agencé entre le premier manchon conducteur et le second manchon conducteur.

12. Mécanisme d'articulation selon la revendication 10 ou 11, dans lequel chacun du premier joint tournant et du second joint tournant est un joint tournant coaxial, et dans lequel la connexion entre le premier joint tournant et le second joint tournant comprend un ou plusieurs coudes (418, 428a, 428b, 448) pour introduire un décalage du second axe de rotation par rapport au premier axe de rotation de sorte que le second axe de rotation soit différent du premier axe de rotation.

13. Mécanisme d'articulation selon la revendication 8 ou 9, dans lequel le premier joint tournant et le second joint tournant sont connectés via une connexion comprenant une microbande, la microbande comprenant des premier et second conducteurs séparés par une couche diélectrique plane.

14. Mécanisme d'articulation selon l'une quelconque des revendications 9 à 13, dans lequel au moins l'un du premier joint tournant et du second joint tournant est un joint selon l'une quelconque des revendications 1 à 8, le premier élément de joint étant le premier substrat plan et le second élément de joint étant le second substrat plan.

15. Mécanisme d'articulation selon l'une quelconque des revendications 9 à 14, comprenant en outre l'un des éléments suivants :
un connecteur permettant de monter un instrument électrochirurgical sur le mécanisme d'articulation ; ou
un instrument électrochirurgical formé d'un seul tenant avec le premier élément de joint ou le second élément de joint du second joint tournant.

16. Dispositif électrochirurgical comprenant
un mécanisme d'articulation selon l'une quelconque des revendications 8 à 15 ; et
un instrument électrochirurgical monté au niveau de l'extrémité distale du mécanisme d'articulation.
